(19) Europäisches Patentamt
European Patent Office
Office européen des brevets

(11) Publication number: 0 547 790 B1

# (12) EUROPEAN PATENT SPECIFICATION

(45) Date of publication of patent specification: 23.08.95

(51) Int. Cl.[6]: A61K 7/13, A61K 7/06

(21) Application number: 92310788.2

(22) Date of filing: 25.11.92

## (54) Dye composition for Keratinous fibers.

(30) Priority: 18.12.91 JP 335166/91

(43) Date of publication of application:
23.06.93 Bulletin 93/25

(45) Publication of the grant of the patent:
23.08.95 Bulletin 95/34

(84) Designated Contracting States:
DE FR GB

(56) References cited:
EP-A- 0 429 855
GB-A- 2 173 515

PARFUMS COSMéTIOUES ARôMES vol. 51, July 1983, PARIS (FRANCE) pages 67 - 72 DR.KOLLMEIER 'les copolymères polysiloxanes polyéthers comme additifs dans lesformulations cosmétiques'

DATABASE WPIL Week 8652, Derwent Publications Ltd., London, GB; AN 83-03088K

(73) Proprietor: Kao Corporation
1-14-10, Nihonbashikayaba-cho,
Chuo-ku
Tokyo (JP)

(72) Inventor: Imamura, Takashi, c/o Kao Corporation
Tokyo Research Laboratories,
2-1-3, Bunka
Sumida-ku,
Tokyo (JP)
Inventor: Murai, Michiko, c/o Kao Corporation
Tokyo Research Laboratories,
2-1-3, Bunka
Sumida-ku,
Tokyo (JP)
Inventor: Shibata, Yutaka, c/o Kao Corporation
Tokyo Research Laboratories,
2-1-3, Bunka
Sumida-ku,
Tokyo (JP)

(74) Representative: Daley, Michael John et al
F.J. CLEVELAND & COMPANY
40/43 Chancery Lane
London, WC2A 1JO (GB)

EP 0 547 790 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a dye composition for keratinous fibers for example human hair, and more particularly to a dye composition for keratinous fibers which is excellent in the dyeing ability and has excellent conditioning effects.

Conventional oxidation-type hair dye compositions are essentially composed of an oxidative dye and an oxidizer, and they involve danger of irritating the skin of the head or giving damages to the hair, if they are not handled properly with care, because hydrogen peroxide is reacted in the presence of an alkali in the course of the hair-dyeing operation. In order to overcome these problems which the oxidation-type hair dye compositions cannot avoid, semi-permanent dye compositions containing a direct dye are proposed as they give less adverse effects to the hair or the skin of the head.

The term "direct dye" throughout the description means the dyes which can express color without aid of other substances. In other words, it encompasses the dyes excepting oxidative dyes which first develop color when oxidized by an oxidizer such as hydrogen peroxide. Examples of the direct dye include nitro dyes, acidic dyes, basic dyes and disperse dyes.

However, these semi-permanent hair dye compositions in general are attended by a drawback. The feel of the hair i.e. its smoothness, softness and ease of finger combing at the time of rinsing and drying becomes worse because of the presence of a relatively great amount of a solvent. Finger combing is the running of the fingers through the hair. Good feel to the touch of the hair during rinsing through drying is important and is required especially for semi-permanent dye compositions which have recently been on the market because their manner of use is similar to that of hair rinses, in other words, applying a hair dye composition to the hair with bare hands or fingers and rinsing off subsequently. Enhanced hair conditioning effects are also demanded in this type of hair dye compositions.

In the meantime, there are many publications in this technical field. For instance, Japanese patent publication (kokoku) No. 55887/1986 discloses a method of providing conditioning effects with the dyed hair using a hair dye composition comprising a silicone derivative such as a polyether modified silicone; Japanese patent publication (kokai) No.157713/1983 discloses a method of providing the hair with conditioning effects which last for a somewhat prolonged period by using a water-soluble cationic polymer and a water-soluble anionic surfactant; British patent No. 21753515 discloses a method of providing the hair with conditioning effects where a direct hair dye, cationic silicone surfactant and a hydroxyl silicone derivative are combined. Any of these methods does not fully meet consumers' needs in terms of conditioning effcts during rinsing and drying.

In particular, it is to be noted that hair dye compositions containing an acid dye, which is a direct dye, are accompanied by a drawback of reduced hair-dyeing ability when quaternary salt type cationic surfactants which are widely used as a hair conditioning agent in hair care products. This is considered to be caused by water-insoluble complexes formed between the acid dye and the quaternary salt type cationic surfactant.

Accordingly, an object of this invention is to provide a dye composition for keratinous fibers comprising a direct dye, and is excellent in the dyeing ability and has excellent conditioning effects.

The above and other objects, features and advantages of this invention will become apparent from the following description.

The present invention is based on the finding that a combination use of a direct dye, a certain polyoxyalkylene modified organopolysiloxane and an organic solvent under a certain pH can provide a dye composition for keratinous fibers which is excellent in the feel to the touch of the hair during rinsing and drying.

According to the present invention, there is provided a dye composition for keratinous fibers characterized by comprising the following ingredients (A), (B) and (C):

(A) a direct dye,

(B) a polyoxyalkylene modified organopolysiloxane which contains 3 to 30% by weight of a polyoxyalkylene group in a molecule thereof, and is dispersible in water,

(C) an organic solvent,

and having a pH of 2.0 to 4.5.

Examples of the direct dye (A) include nitro dyes such as 3-amino-4-hydroxynitrobenzene, 2-amino-5-hydroxynitrobenzene, 2-amino-3-hydroxynitrobenzene, 2-amino-5-N,N-bis-beta-hydroxyethylaminonitrobenzene, 2-amino-4-chloro-5-N-beta-hydroxyethylaminonitrobenzene, 2-amino-4-methyl-5-N-beta-hydroxyethylaminonitrobenzene, 3,4-bis-(N-beta-hydroxyethylamino)nitrobenzene, 2-amino-4-methyl-5-N-beta, gamma-dihydroxypropylaminonitrobenzene, 2-amino-4-methyl-5-beta-aminoethylaminonitrobenzene, 2-amino-4-hydroxynitrobenzene, and in particular 3,4-diaminonitrobenzene, 2,5-diaminonitrobenzene, 2-amino-5-N-beta-hydroxyethylaminonitrobenzene, 2-N-beta-hydroxyethylamino-5-N,N-bis-beta-hydroxyethylaminonitrobenzene, 2-N-methylamino-5-N,N-bis(beta-hydroxyethyl)aminonitrobenzene, 2-N-methylamino-5-N-methyl-N-beta-hydroxyethylaminonitrobenzene, 2-N-beta-hydroxyethylamino-5-hydroxynitrobenzene, 3-methoxy-4-N-beta-hydroxyethylaminonitrobenzene, 4-nitro-3-methylaminophenoxy ethanol, 2-N-beta-hydroxyethylamino-5-aminonitrobenzene, 2-N-beta-hydroxyethylaminonitrobenzene, 3-amino-4-N-beta-hydroxyethylaminonitrobenzene, 3-beta-hydroxyethyoxy-4-N-beta-hydroxyethylaminoÜitrobenzene, 2-amino-5-N-methylaminonitrobenzene, 2-amino-3-methylnitrobenzene, 2-N-beta-hydroxyethylamino-5-beta, gamma-dihydroxypropyloxynitrobenzene, 3-hydroxy-4-N-beta-hydroxyethylaminonitrobenzene, 3-hydroxy-4-aminonitrobenzene, 2,5-N,N'-beta-hydroxyethylaminonitrobenzene, 2-N-methylamino-4-o-beta, gamma-dihydroxypropyloxynitrobenzene, 2-N-beta-aminoethylamino-5,N,N-bis-(beta-hydroxyethyl)aminonitrobenzene, 2-N-beta-aminoethylamino-4-methoxynitrobenzene, 1-amino-4-methylaminoanthraquinone, 1,4-diaminoanthraquinone; acid dyes such as C.I. Acid Red 27, C.I. Acid Red 51, C.I. Acid Red 18, C.I. Acid Red 92, C.I. Acid Red 94, C.I. Acid Red 52, C.I. Acid Yellow 23, C.I. Food Yellow 3, C.I. Food Green 3, C.I. Food Blue 2, C.I. Acid Blue 74, C.I. Pigment Red 57-1, C.I. Acid Red 33, C.I. Acid Red 87, C.I. Acid Red 92, C.I. Acid Red 94, C.I. Acid Orange 7, C.I. Acid Red 95, C.I. Acid Yellow 73, C.I. Acid Yellow 3, C.I. Acid Green 25, C.I. Solvent Green 7, C.I. Acid Green 5, C.I. Acid Blue 5, C.I. Acid Blue 9, C.I. Acid Orange 24, C.I. Acid Violet 9, C.I. Food Red 6, C.I. Acid Red 26, C.I. Food Red 1, C.I. Acid Red 88, C.I. Acid Orange 20, C.I. Acid Yellow 40, C.I. Acid Yellow 1, C.I. Acid Yellow 36, C.I. Acid Yellow 11, C.I. Acid Green 1, C.I. Acid Green 3, C.I. Acid Violet 43 and C.I. Acid Black 1; oil-solble dyes such as C.I. Solvent Red 49, C.I. Solvent Red 48, C.I. Solvent Red 43, C.I. Solvent Red 72, C.I. Solvent Red 73, C.I. Acid Yellow 73, C.I. Solvent Yellow 33, C.I. Solvent Green 3, C.I. Solvent Violet 13, C.I. Solvent Red 24, C.I. Solvent Orange 7, C.I. Solvent Orange 2, C.I. Solvent Yellow 5, C.I. Solvent Yellow 6 and C.I. Solvent Blue 63; disperse dyes such as C.I. Solvent Red 49, C.I. Solvent Red 48, C.I. Solvent Red 43, C.I. Solvent red 23, C.I. Solvent Red 72, C.I. Solvent Red 73, C.I. Acid Yellow 73, C.I. Solvent Yellow 33, C.I. Solvent Green 3, C.I. Solvent Violet 13, C.I. Solvent Red 24, C.I. Solvent Orange 7, C.I. Solvent Yellow 5, C.I. Solvent Yellow 6 and C.I. Solvent Blue 63; basic dyes such as C.I. base violet and C.I. Solvent Red 49; basic dyes manufactured by Williams Co., such as Sienna Brown, Mahogany, Madder Red, Steel Blue and Straw Yellow; disperse dyes such as Disperse Black 9, Disperse Blue 1, Disperse Blue 3, Disperse Violet 1 and Disperse Violet 4.

For preparing dye compositions according to this invention in a so- called rinse type which can be used by bare hands or fingers, the following direct acid dyes are preferred: C.I. Acid Yellow 23, C.I. Solvent Green 7, C.I. Acid Red 27, C.I. Acid Red 18, C.I. Food Green 3, C.I. Food Blue 2, C.I. Acid Blue 9, C.I. Acid Yellow 1, C.I. Acid Red 52, C.I. Pigment Red 57-1, C.I. Acid Orange 7, C.I. Acid Black 1, C.I. Acid Green 25, C.I. Acid Violet 43, in particular, C.I. Acid Black 1, C.I. Acid Violet 43, C.I. Acid Orange 7, C.I. Acid Yellow 1 and C.I. Acid red 52.

These direct dyes are employed singly or as a mixture of two or more. They are incorporated into the dye compositions according to the present invention in an amount of 0.02 to 5% by weight (hereinafter may be referred to %), and more preferably 0.02 to 1% based on the total amount of the dye composition. An amount less than 0.01% cannot achieve a sufficient dyeing effect, and amount exceeding 5% will cause significant staining of the skin of hands and fingers, thus not practical. In the case where the dye compositions of this invention are formed into rinse-type compositions which can be handled by bare hands or fingers, the amount of the dyes is preferably 0.02 to 0.1% in view of the balance between the dyeing performance and ignorable staining to the skin.

Ingredient (B) is a polyoxyalkylene modified organo-polysiloxane which contains 3 to 30% by weight of a polyoxyalkylene group in a molecule thereof and is dispersible in water as mentioned before. Polyoxyalkylene groups may be present at any position of the polysiloxane chain, and the following structures (B-1) to (B-4) may be mentioned.

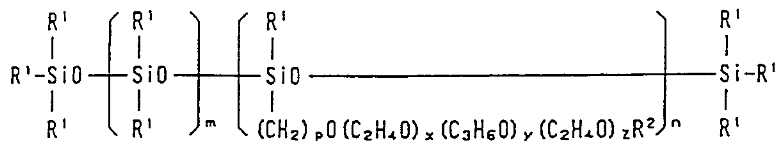

(B-1)

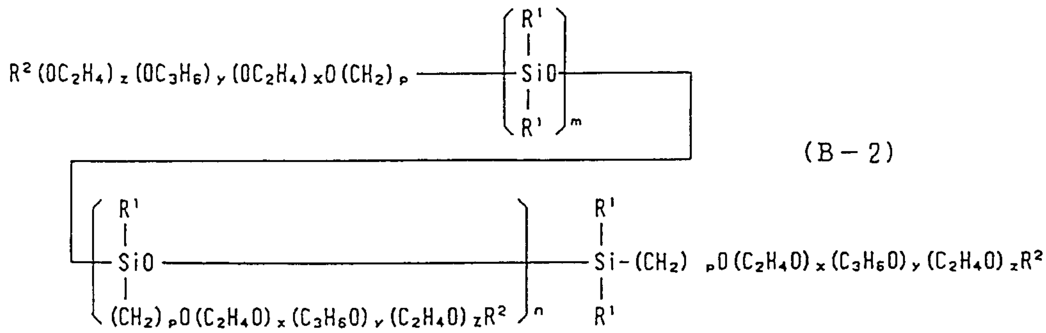

(B-2)

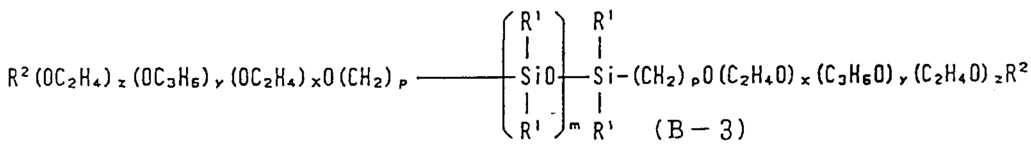

(B-3)

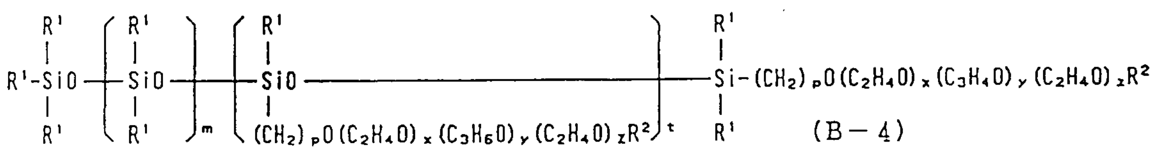

(B-4)

wherein $R^1$ is a C1 to C3 alkyl or phenyl group, $R^2$ is a hydrogen atom or a C1 to C12 alkyl group, mis an integer of 20 to 200, n is an integer of 1 to 10, x is an integer of 0 to 15, y is an integer of 0 to 10, z is an integer of 0 to 15, p is an integer of 1 to 5, t is an integer of 0 to 10, provided the sum of $x+y+z$ is 1 or more but 20 or less.

The proportion by weight of of the polyoxyalkylene group contained in a molecule of the polyoxyalkylene modified organopolysiloxane (B) is preferably from 3 to 30%, and more preferably 5 to 25%. A proportion less than 3% cannot achieve excellent conditioning effects because the polyoxyalkylene modified organopolysiloxane will become difficult to disperse in water. Further, users will feel friction at the time of rinsing the hair. On the other hand, proportion exceeding 30% will make the modified polysiloxane easy to dissolve in water such as rinse water, which eventually provides reduced conditioning effects because it is readily rinsed off with water.

The polyoxyalkylene modified organopolysiloxanes which are useful in the present invention is capable of being dispersed in water of 25 °C, providing no or very much reduced frictional feel to the touch of the hair at the time of rinsing while imparting excellent conditioning effects at the time of drying the hair, that is, no or very much reduced frictional feel to the touch of the hair and proper and agreeable smoothness and softness.

Here, the polyoxyalkylene modified organopolysiloxanes which are capable of being dispersed in water according to this invention are such that an aqueous 1% solution of the modified organopolysiloxanes cannot transparently dissolve and observed as turbid by the naked eye. Part of the modified organopolysiloxanes may float on the surface or settle, which is also within the scope of this invention.

These polyoxyalkylene modified organopolysiloxanes are employed singly or as a mixture of two or more. The amount of the modified organopolysiloxanes are preferably 0.01 to 10%, more preferably from 0.1 to 4% based on the total amount of the dye composition of this invention. An amount less than 0.01% cannot achieve a sufficient hair conditioning effect, while an amount exceeding 10% will make the composition sticky thus not favorable.

The dye compositions according to the present invention can be formed into ordinary forms of conventional hair dye compositions. That is, a hair dye composition is applied onto karatinous fibers such as the hair by using gloves, etc., allowed to stand for a certain period of time and subsequently rinsed off. Another form of the dye compositions of this invention, which is more preferable, is that of a hair rinse-type. In this case, a composition is directly applied to the hair or the like with bare hands or fingers, allowed to stand for a short time of from 30 to 300 seconds, and subsequently rinsed off. In the latter case, the dyeing effects are visually acknowledged upon repeated use of 5 to 10 times in general, which might be varied depending on the type of the hair fiber and manner of use.

The organic solvents (C) which are useful in this invention include the compounds represented by formula (C-1) below, C1 to C4 alkylene carbonates, and N-alkylpyrrolidones represented by formula (C-2):

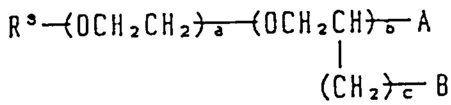

(C - 1)

wherein $R^3$ is a hydrogen atom, a lower alkyl group or a group

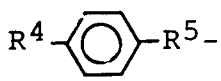

wherein $R^4$ is a hydrogen atom, methyl group or a methoxy group, $R^5$ is a hand for bonding or a C1 - C3 saturated or unsaturated divalent hydrocarbon, A and B independently represent a hydrogen atom or a hydroxyl group, a, b and c independently represent an integer of 0 to 5, excepting the two cases where $a = b = c = 0$ and $A = H$, and $a = b = c = 0$, $R^3 = H$ and $A = OH$;

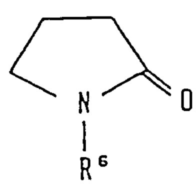

(C - 2)

wherein $R^6$ is a C1 - C18 linear or branched alkyl group.

Examples of the organic solvents according to the present invention include ethanol, isopropanol, n-propanol, n-butanol, isobutanol, ethylene glycol, propylene glycol, isoprene glycol, 1,3-butanediol, benzyl alcohol, cinnamic alcohol, phenethyl alcohol, p-anisyl alcohol, p = methylbenzyl-alcohol, phenoxyethanol, 2-benzyloxy ethanol, methyl carbitol, ethyl carbitol, propyl carbitol, butyl carbitol, triethylene glycol monoethylether, triethylene glycol monobutyl ether, glycerol, N-methylpyrrolidone, N-octylpyrrolidone, N-laurylpyrrolidone and the like. It is preferred that these organic solvents (C) be incorporated into the dye composition of this invention in an amount of from 0.5 to 50%, preferably 1 to 35% based on the total weight of the composition. An amount less than 0.5% cannot achieve a sufficient dyeing performance in a short time of contact of the composition with the hair in case where a "rinse-type" dye composition to be handled with bare hands or fingers is prepared. An amount exceeding 50% is not advisable either, because improvements of the effects will no more be expected.

The pH of the dye compositions according to the present invention as measured on an aqueous 10% solution of the composition is preferably from 2.0 to 4.5, more preferably from 2.5 to 4.5 and most preferably from 2.5 to 4.0. When the compositions are formed into a rinse type, where bare hands or fingers come into direct contact with the compositions, a pH exceeding 4.5 is not advisable because the time during which the compositions are in contact with the hair cannot be shortened, while on the other hand, a

pH lower than 2.0 raises a problem of irritating the skin due to its acidity.

It is preferred that the compositions according to this invention be so formulated that the buffer index of an aqueous 10% solution of the compositions is from 0.01 to 0.2 gram equivalent/litre, in view of the reduced contact time after application of the compositions to the hair. Here the buffer index is a value calculated from the following equation, factoring the concentration of the base required for elevating the pH of an aqueous 10% solution of the dye composition at 25 °C by value 1 frm the initial value.

$$\text{Buffer Index} = \left| \frac{dC_B}{dpH} \right|$$

wherein $C_B$ is an ion concentration of a base (gram equivalent/litre).

A buffer index less than 0.01 gram equivalent/litre cannot achieve a sufficient dyeing effect in a short time of contact, whereas an index value exceeding 0.2 gram equivalent/litre cannot improve the dyeing effect remarkably. Also, the pH buffering agent or other ingredients to be incorporated are difficult to dissolve in the composition, thus not preferable. Preferable range of the buffer index, therefore, is 0.01 to 0.05 gram equivalent/litre.

The buffer capability according to the invention can be given to the composition by adding thereto pH buffer agents, surfactants, chelating agents, preservatives and the like. Among them, pH buffering agents are those showing the buffer action in a pH range of pH 2.0 to 4.5 and selected from organic acids, inorganic acids and/or their salts. Examples of the organic acids include citric acid, glycolic acid, succinic acid, tartaric acid, lactic acid, fumaric acid, malic acid, levulinic acid, butyric acid, valeric acid, oxalic acid, maleic acid and mandelic acid. Examples of the inorganic acids include phosphoric acid, sulfuric acid and nitric acid. Examples of the salts of these acids include sodium salts, potassium salts, ammonium salts and alkanolamine salts such as triethanolamine salts. The amount of the buffer compounds to be incorporated is not particularly limited and depends on the compounds used. For instance, when a sodium salt of citric acid is used as a buffer compound, the concentration of the salt is higher than about 2 - 2.5 wt %.

The hair dye compositions according to this invention may contain optional ingredients such as surfactants; cationic polymers; oils; viscosity modifiers such as hydroxyethylcellulose and xanthane gum; silicone derivatives other than the mentioned ingredients (B); perfumes; preservatives; UV absorbers; antioxidants; bactericides; pearlescent agents; opacity agents and the like as long as they will not impede the effects of the present invention. Here, examples of surfactants include anionic surfactants such as olefin sulfonic acid, alkane sulfonic acid, aliphatic alkyl ether carboxylic acid and N-acylamino acid; amphoteric surfactants such as amidobetaine, carbobetaine and hydroxysulfobetaine; cationic surfactants such as mono- or di- alkyl quaternary ammonium salts; and nonionic surfactants such as polyoxy-alkylene alkyl ethers. Examples of cationic polymers include cationized cellulose, catinonized starch, cationized guar gum, polymers of diallyl quaternary ammonium salts, copolymers of diallyl quaternary ammonium salt/acrylic amide and copolymers of hydroxyethylcellulose/dimethyl diallyl quaternary ammonium salt. Examples of oils include aliphatic esters, linear or branched alkyl glyceryl ethers and branched higher alcohols.

The dye compositions for keratinous fibers according to the present invention are prepared by any known processes, by blending the aforementioned ingredients. The manner of use of the compositions is described hereinbefore.

The dye compositions of this invention are excellent in the dyeing performance and in the conditioning effects providing very much favorable feel to the touch of the hair at the time of rinsing and drying the hair.

## Examples

The present invention will now be described in more detail by way of examples, but they should not be construed as limiting the invention thereto.

Examples 1 to 3:

About 10 g of dry white hair fibers were shampooed, drained, and uniformly and quickly applied with 3 g of a composition in Table 1, allowed to stand for 30 seconds at 35 °C, followed by rinsing and drying. A panel consisting of 10 expert members evaluated each composition with respect to the following items during rinsing and drying the hair. The results are shown in Table 1.

Evaluations on conditioning effects:

a) Finger Combing during Rinsing:

A: No frictional feel with excellent smooth finger combing
B: Reduced dgree of frictional feel with moderate finger combing
C: Certain degree of frictional feel with unfavorable finger combing
D: Very frictional feel with catching

b) Softness of the hair during rinsing and drying:

A: Very soft and resilient
B: Soft
C: Lack of softness

c) Combing at the time of drying the hair:

A: Easy combing and smooth
B: A little catching when combing

C: Catching especially at the end of hair fibers

Table 1

(% by weight)

| | Compara. Compn. | | | Invention Compn. | | |
|---|---|---|---|---|---|---|
| | 1 | 2 | 3 | 1 | 2 | 3 |
| (1)Ethanol (%) | 25.0 | 25.0 | 25.0 | 25.0 | 25.0 | 25.0 |
| (2)Benzyl alcohol (%) | 5.0 | 5.0 | 5.0 | 5.0 | 5.0 | 5.0 |
| (3)Citric acid (%) | 0.5 | 0.5 | 0.5 | 0.5 | - | 3.0 |
| (4)C.I. Acid Black 1 (%) | - | - | - | - | 0.03 | - |
| (5)C.I. Acid Violet 43 (%) | - | - | - | - | 0.03 | - |
| (6)C.I. Acid Orange 7 (%) | 0.05 | 0.05 | 0.05 | 0.05 | - | 0.05 |
| (7)Polyoxyethylene-modified organopolysiloxane (See note 1) | - | - | - | 1.0 | 1.0 | 2.5 |
| (8)Polyoxyethylene modified organopolysiloxane (See note 2) | - | - | 1.0 | - | - | - |
| (9)Cationized Cellulose (See note 3) | 0.5 | - | - | - | - | - |
| (10)Hydroxyethylcellulose | 1.5 | 1.5 | 1.5 | 1.5 | 1.5 | 1.5 |
| (11) NaOH | ← Suitable Amount (See note 4) → | | | | | |
| (12) Water | ← Balance → | | | | | |
| Finger combing during rinsing | C | D | C | B | B | A |
| Softness of the hair during rinsing and drying | B | C | C | A | A | A |
| Combing at the time of drying the hair | C | C | B | A | A | A |

note 1) Polyoxyethylene group: 20 wt.%; MW: about 7500; Formula (B-1) where $R^1=CH_3$, $R^2=H$, M=70 to 80, n= 2 to 4, x= 10 to 12, y=z=0, p=3.

note 2) Polyoxyethylene group: 50 wt.%; Formula (B-1) where $R^1=CH_3$, $R^2=H$.

note 3) Polymer JR400 manufactured by UCC Co.

note 4) Amount required for adjusting the pH to 4.0.

Example 4:

Ten female monitors in their forties used the invention composition 3 (see Table 1) in the following manner. About 10% of their front hair was white. Beauticians shampooed the monitors' hair and applied about 15 g of the composition. The composition was allowed to stand for 60 seconds at 35 °C, rinsed and dried with a hair drier. This cycle of operation was repeated for four times, and conspicuousness of white hair was visually evaluated by a panel consisitng of ten members. The results are shown in Table 2.

Table 2

| | No change in conspicuousness of white hair | Conspicuousness of white hair is somewhat mitigated | White hair is no more conspicuous |
|---|---|---|---|
| Invention composition 3 | 13 | 65 | 22 |

Note) The change in the color of the hair of the monitors before and after the use of the composition was observed by panel members, and classified into 3 groups of "No change in conspicuousness of white hair", "Conspicuousness of white hair is somewhat mitigated" and "White hairs are no more conspicuous". Ten panel members evaluated the results of 10 monitors, accordingly, the total number of data is 100.

Example 5:

A composition having the following formulation was prepared. The composition was applied to a sample of Caucasian blond hair (2.5 g/5 g of hair) and allowed to stand for 30 minutes at 30 °C, followed by rinsing with running water and drying. The feel to the touch of the hair during rinsing and drying was very good.

| (Formulation) | | |
|---|---|---|
| (1) | C.I. Steel Blue | 0.025 (%) |
| (2) | 2-Amino-5-beta-N-hydroxyethylaminonitrobenzene | 0.050 |
| (3) | Trimethyl ammonium behenyl chloride | 1.0 |
| (4) | Cetostearyl alcohol | 4.0 |
| (5) | Propylene glycol | 7.0 |
| (6) | Polyoxyethylene modified organopolysiloxane (Polyoxyethylene group: 20 wt.%; MW: about 5500; Formula B-1 where $R^1 = CH_3$, $R^2 = H$, m = 50 -55, n = 2 - 3, x = 9 - 11, y = z = 0, p = 3) | 2.0 |
| | NaOH | Suitable Amount (Adjusted to pH.4.3) |
| (8) | Phosphoric acid | Suitable Amount (Adjusted to pH 4.3) |
| (9) | Water | Balance |

Example 6:

The similar procedures were followed as described in Example 5 using the following composition. The feel to the touch of the hair during rinsing and drying was very good.

| (Formulation) | | |
|---|---|---|
| (1) | 1,3-Butylene glycol | 20.0 (%) |
| (2) | 2-Benzyloxyethanol | 7.0 |
| (3) | Lactic Acid | 2.0 |
| (4) | C.I. Acid Black 1 | 0.02 |
| (5) | C.I. Acid violet 43 | 0.02 |
| (6) | C.I. Acid Orange 7 | 0.04 |
| (7) | Polyoxyethylene modified organo-polysiloxane (Polyoxyethylene group: 18 wt.%; MW: about 6700; Formula B-2 where $R^1$=$CH_3$, $R^2$=H, m=65 - 75, n=1, x=8 - 10, y=z=0, p=3) | 1.5 |
| (8) | Hydroxyethylcellulose | 1.5 |
| (9) | NaOH | Suitable Amount (Adjusted to pH3.5) |

Example 7:

The similar procedures were followed as described in Example 5 using the following composition. The feel to the touch of the hair during rinsing and drying was very good.

| (Formulation) | | |
|---|---|---|
| (1) | Ethanol | 20.0 (%) |
| (2) | Benzyl alcohol | 7.0 |
| (3) | Lactic Acid | 2.0 |
| (4) | C.I. Acid Black 1 | 0.01 |
| (5) | C.I. Acid Violet 43 | 0.01 |
| (6) | C.I. Acid Red 33 | 0.03 |
| (7) | Polyoxyethylene modified organopolysiloxane (Polyoxyethylen group: 7 wt.%; MW: about 13000; Formula B-3, where $R^1$ = $CH_3$, $R^2$ = H, m = 150 - 170, x = 8 -12, y = z = 0, p = 3) | 2.5 |
| (8) | Xanthan gum | 1.0 |
| (9) | NaOH | Suitable Amount (Adjusted to pH 3.0) |

Example 8:

The similar procedures were followed as described in Example 5 using the following composition. The feel to the touch of the hair during rinsing and drying was very good.

| (Formulation) | | |
|---|---|---|
| (1) | Ethanol | 20.0 (%) |
| (2) | Benzyl alcohol | 3.0 |
| (3) | Citric acid | 2.0 |
| (4) | C.I. Acid Black 1 | 0.01 |
| (5) | C.I. Acid Violet 43 | 0.01 |
| (6) | C.I. Acid Red 33 | 0.03 |
| (7) | Polyoxyethylene modified organopolysiloxane (Polyoxyethylene group: 19 wt.%; MW: about 4200; Formula B-1, where $R^1 = CH_3$, $R^2 = H$, m = 35-45, n = 1-3, x = 8-10, y = z = o, p = 3) | 0.5 |
| (8) | Hydroxyethylcellulose | 1.5 |
| (9) | NaOH | Suitable Amount (Adjusted to pH 4.0) |

**Claims**

**1.** A dye composition for keratinous fibers characterized by comprising the following ingredients (A), (B) and (C):
(A) a direct dye,
(B) a polyoxyalkylene modified organopolysiloxane which contains 3 to 30% by weight of a polyoxyalkylene group in a molecule thereof, and is dispersible in water,
(C) an organic solvent,
and having a PH of 2.0 to 4.5.

**2.** A dye composition according to Claim 1, wherein the proportions of the ingredients (A), (B) and (C) are
(A) 0.01 to 5% by weight,
(B) 0.01 to 10% by weight and
(C) 0.5 to 50% by weight
based on the total weight of the composition.

**3.** A dye composition according to Claim 1, wherein the proportions of the ingredients (A), (B) and (C) are
(A) 0.02 to 0.1% by weight,
(B) 0.1 to 4% by weight,
(C) 1 to 35% by weight
based on the total weight of the composition.

**4.** A dye composition according to any of the preceding claims, wherein the direct dye (A) is an acid dye.

**5.** A dye composition according to any of claims 1 to 3, wherein the direct dye (A) is selected from the group consisting of C.I. Acid Yellow 23, C.I. Solvent Green 7, C.I. Acid Red 27, C.I. Acid Red 18, C.I. Food Green 3, C.I. Food Blue 2, C.I. Acid Blue 9, C.I. Acid Yellow 1, C.I. Acid Red 52, C.I. Pigment Red 57-1, C.I. Acid Orange 7, C.I. Acid Black 1, C.I. Acid Green 25 and Acid Violet 43.

**6.** A dye composition according to any of the preceding claims, wherein the polyoxyalkylene amodified organopolysiloxane (B) is selected from the group consisting of (B-1), (B-2), (B-3) and (B-4):

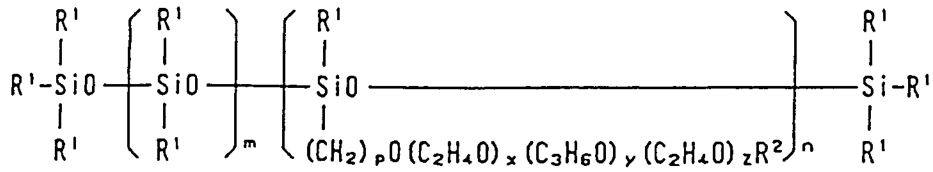

(B－1)

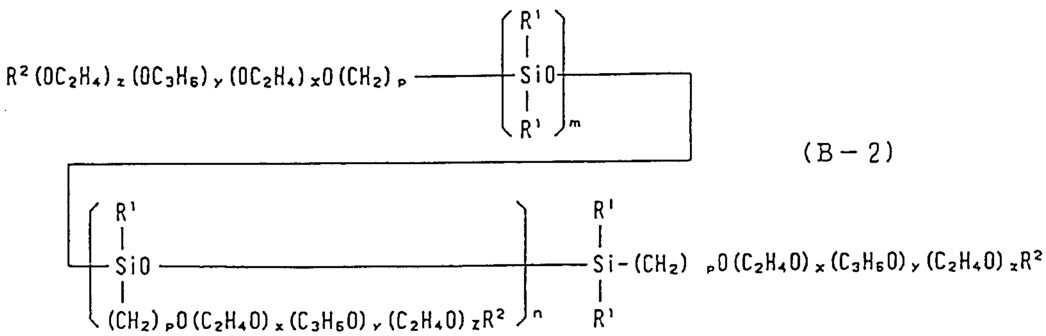

(B－2)

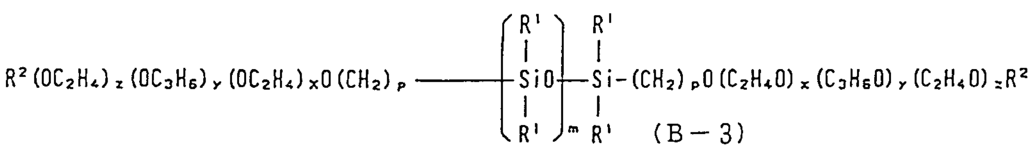

(B－3)

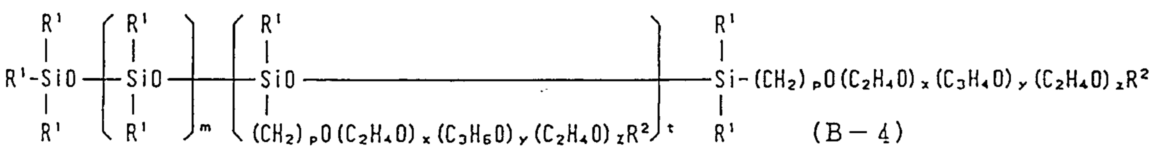

(B－4)

wherein $R^1$ is a C1 to C3 alkyl or phenyl group, $R^2$ is a hydrogen atom or a C1 to C12 alkyl group, mis an integer of 20 to 200, n is an integer of 1 to 10, x is an integer of 0 to 15, y is an integer of 0 to 10, z is an integer of 0 to 15, p is an integer of 1 to 5, t is an integer of 0 to 10, provided the sum of x+y+z is 1 or more but 20 or less.

7. A dye composition according to any of the preceding claims, wherein the organic solvent (C) is selected from the group consisting of the compounds represented by formula (C-1), N-alkylpyrrolidones represented by formula (C-2), and C1 to C4 alkylene carbonates:

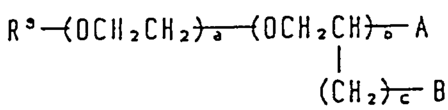

(C－1)

wherein $R^3$ is a hydrogen atom, a lower alkyl group or a group

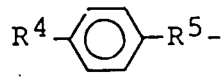

wherein $R^4$ is a hydrogen atom, methyl group or a methoxy group, $R^5$ is a hand for bonding or a C1 - C3 saturated or unsaturated divalent hydrocarbon, A and B independently represent a hydrogen atom or a hydroxyl group, a, b and c independently represent an integer of 0 to 5, excepting the two cases where a = b = c = 0 and A = H, and a = b = c = 0, $R^3$ = H and A = OH;

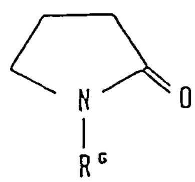

(C - 2)

wherein $R^6$ is a C1 - C18 linear or branched alkyl group.

**8.** A dye composition according to any of claims 1 to 6, wherein the organic solvent (C) is selected from the group consisting of ethanol, isopropanol, n-propanol, n-butanol, isobutanol, ethylene glycol, propylene glycol, isoprene glycol, 1,3-butanediol, benzyl alcohol, cinnamic alcohol, phenethyl alcohol, p-anisyl alcohol, p = methylbenzyl-alcohol, phenoxyethanol, 2-benzyloxy ethanol, methyl carbitol, ethyl carbitol, propyl carbitol, butyl carbitol, triethylene glycol monoethylether, triethylene glycol monobutyl ether, glycerol, N-methylpyrrolidone, N-octylpyrrolidone and N-laurylpyrrolidone,

**9.** A dye composition according to any of the preceding claims, wherein the pH falls in the range of from 2.5 to 4.5.

**10.** A dye composition according to any of the preceding claims, wherein the buffer index of an aqueous 10% solution of said dye composition is 0.01 to 0.2 gram equivalent/litre.

**11.** A due composition according to any of the preceding claims, which is directly applied to the hair with bare hands or fingers, allowed to stand for 30 to 300 seconds and is subsequently rinsed off with water.

**12.** A method of dyeing the hair characterized by applying a composition comprising the following ingredients (A), (B) and (C),
(A) a direct dye,
(B) a polyoxyalkylene modified organopolysiloxane which contains 3 to 30% by weight of a polyoxyalkylene group in a molecule thereof, and is dispersible in water,
(C) an organic solvent,
and having a pH of 2.0 to 4.5, directly to the hair with bare hands or fingers, allowing to stand for 30 to 300 seconds and then rinsing off with water.

## Patentansprüche

**1.** Färbemittel-Zubereitung für Keratin-Fasern, dadurch gekennzeichnet, daß sie die folgenden Bestandteile (A), (B) und (C) umfaßt:
(A) einen Direkt-Farbstoff;
(B) ein mit einer Polyoxyalkylen-Gruppe modifiziertes Organopolysiloxan, welches 3 bis 30 Gew.-% einer Polyoxyalkylen-Gruppe im Molekül enthält und in Wasser dispergierbar ist;
(C) ein organisches Lösungsmittel;
und die einen pH-Wert von 2,0 bis 4,5 aufweist.

**2.** Färbemittel-Zubereitung nach Anspruch 1, worin die Mengenanteile der Bestandteile (A), (B) und (C) sind:
(A) 0,01 bis 5 Gew.-%;
(B) 0,01 bis 10 Gew.-%;

(C) 0,05 bis 50 Gew.-%;
bezogen auf das Gesamtgewicht der Zubereitung.

**3.** Färbemittel-Zubereitung nach Anspruch 1, worin die Mengenanteile der Besandteile (A), (B) und (C) sind:
(A) 0,02 bis 0,1 Gew.-%;
(B) 0,1 bis 4 Gew.-%;
(C) 1 bis 35 Gew.-%,
bezogen auf das Gesamtgewicht der Zubereitung

**4.** Färbemittel-Zubereitung nach einem der vorgangehenden Ansprüche, worin der Direktfarbstoff (A) ein saurer Farbstoff ist.

**5.** Färbemittel-Zubereitung nach einem der Ansprüche 1 bis 3, worin der Direkt-Farbstoff (A) gewählt ist aus der Gruppe, die besteht aus C.I. Sauer-Gelb 23 (Acid Yellow 23), C.I. Lösungsmittel-Grün 7 (Solvent Green 7), C.I. Sauer-Rot 27 (Acid Red 27), C.I. Sauer-Rot 18 (Acid Red 18), C.I. Lebensmittel-Grün 3 (Food Green 3), C.I. Lebensattel-Blau 2 (Food Blue 2), C.I. Sauer-Blau 9 (Acid Blue 9), C.I. Sauer-Gelb 1 (Acid Yellow 1), C.I. Sauer-Rot 52 (Acid Red 52), C.I. Pigment-Rot 57-1 (Pigment Red 57-1), C.I. Sauer-Orange 7 (Acid Orange 7), C.I. Sauer-Schwarz 1 (Acid Black 1), C.I. Sauer-Grün 25 (Acid Green 25 und C.I. Sauer-Violett 43 (Acid Violet 43).

**6.** Färbemittel-Zubereitung nach einem der vorangehenden Ansprüche, worin das mit einer Polyalkylen-Gruppe modifizierte Organopolysiloxan (B) gewählt ist aus der Gruppe, die besteht aus den Verbindungen (B-1), (B-2), (B-3) und (B-4):

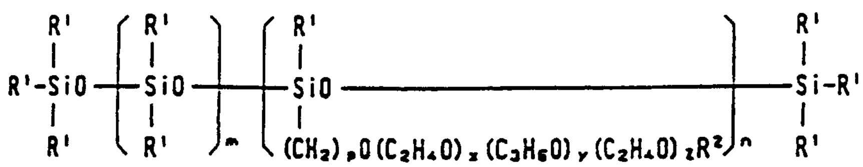
(B−1)

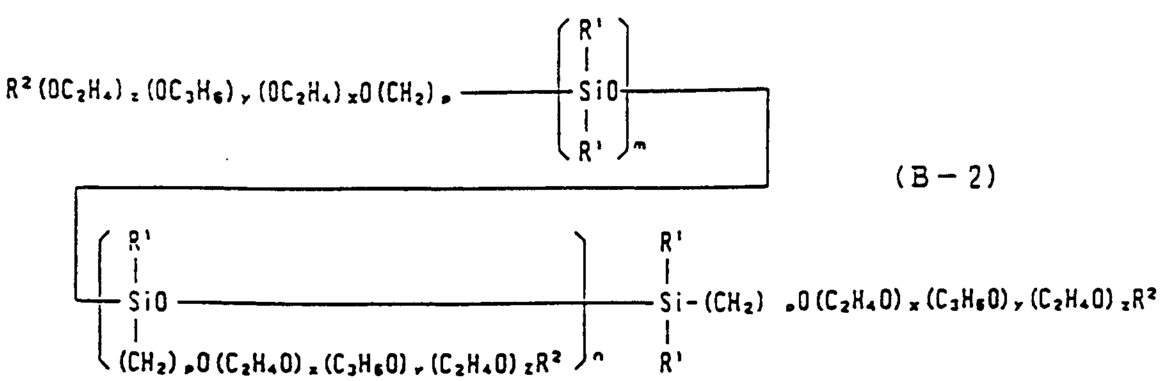
(B−2)

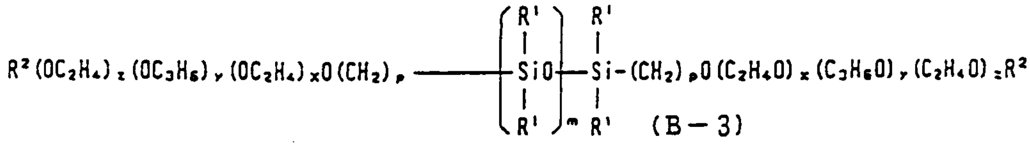

(B − 3)

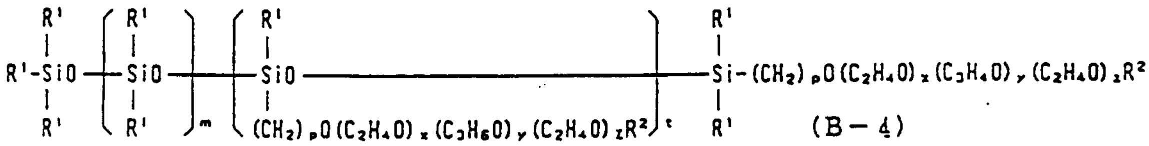

(B − 4)

worin $R^1$ für eine $C_1$- bis $C_3$-Alkyl-Gruppe oder Phenyl-Gruppe steht, $R^2$ für ein Wasserstoffatom oder eine $C_1$- bis $C_{12}$-Alkyl-Gruppe steht, m eine ganze Zahl von 20 bis 200 ist, n eine ganze Zahl von 1 bis 10 ist, x eine ganze Zahl von 0 bis 15 ist, y eine ganze Zahl von 0 bis 10 ist, z eine ganze Zahl von 0 bis 15 ist, p eine ganze Zahl von 1 bis 5 ist, t eine ganze Zahl von 0 bis 10 ist, vorausgesetzt, daß die Summe (x + y + z) 1 oder mehr ist, jedoch 20 oder weniger ist.

**7.** Färbemittel-Zubereitung nach einem der vorangehenden Ansprüche, worin das organische Lösungsmittel (C) gewählt ist aus der Gruppe, die besteht aus Verbindungen, die wiedergegeben werden durch die Formel (C-1), N-Alkylpyrrolidonen, die durch die Formel (C-2) wiedergegeben sind, und C1- bis C4-Alkylencarbonaten:

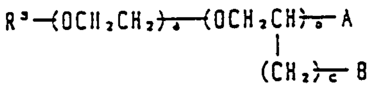

(C − 1)

worin $R^3$ für ein Wasserstoffatom, eine Niederalkyl-Gruppe oder eine Gruppe

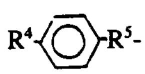

steht, worin $R^4$ für ein Wasserstoffatom, eine Methylgruppe oder eine Methoxy-Gruppe steht und $R^5$ eine Stelle für eine Bindung oder ein gesättigter oder ungesättigter zweiwertiger $C_1$-bis $C_3$- Kohlenwasserstoff-Rest ist, A und B unabhängig voneinander für ein Wasserstoffatom oder eine Hydroxyl-Gruppe stehen, a, b und c unabhängig voneinander für eine ganze Zahl von 0 bis 5 stehen, ausgenommen die beiden Fälle, in denen a = b = c = 0 und A = H sind und a = b = c = 0, $R^3$ = H und A = OH sind;

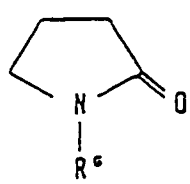

(C − 2)

worin $R^6$ für eine lineare oder verzweigte $C_1$- bis $C_{18}$- Alkyl-Gruppe steht.

**8.** Färbemittel-Zubereitung nach einem der Ansprüche 1 bis 6, worin das organische Lösungsmittel (C) gewählt ist aus der Gruppe, die besteht aus Ethanol, Isopropanol, n-Propanol, n-Butanol, Isobutanol, Ethylenglykol, Propylenglykol, Isoprenglykol, 1,3-Butandiol, Benzylalkohol, Zimtalkohol, Phenethylalkohol, p-Anisylalkohol, p-Methylbenzylalkohol, Phenoxyethanol, 2-Benzyloxyethanol, Methylcarbitol, Ethylcarbitol, Propylcarbitol, Butylcarbitol, Triethylenglykolmonoethylether, Triethylenglykolmonobutylether, Glycerin, N-Methylpyrrolidon, N-Octylpyrrolidon und N-Laurylpyrrolidon.

**9.** Färbemittel-Zubereitung nach einem der vorangehenden Ansprüche, worin der pH-Wert in den Bereich von 2,5 bis 4,5 fällt.

**10.** Färbemittel-Zubereitung nach einem der vorangehenden Ansprüche, worin der Pufferindex einer wäßrigen, 10 %igen Lösung der Färbemittel-Zubereitung 0,01 bis 0,2 Grammequivalente/Liter ist.

**11.** Färbemittel-Zubereitung nach einem der vorangehenden Ansprüche, welche mit nackten Händen oder Fingern unmittelbar auf das Haar aufgebracht wird, 30 bis 300 Sekunden darin belassen wird und danach mit Wasser ausgespült wird.

**12.** Verfahren zum Färben der Haare dadurch gekennzeichnet, daß man eine Zubereitung, die die folgenden Komponenten (A), (B) und (C) umfaßt
(A) einen Direkt-Farbstoff;
(B) ein mit einer Polyoxyalkylen-Gruppe modifiziertes Organopolysiloxan, welches 3 bis 30 Gew.-% einer Polyoxyalkylen-Gruppe im Molekül enthält und in Wasser dispergierbar ist;
(C) ein organisches Lösungsmittel;
und einen pH-Wert von 2,0 bis 4,5 aufweist, mit nackten Händen oder Fingern unmittelbar auf das Haar aufbringt, sie dort 30 bis 300 Sekunden lang beläßt und sie anschließend mit Wasser ausspült.

**Revendications**

**1.** Une composition tinctoriale pour fibres kératiniques caractérisée en ce qu'elle comprend les ingrédients (A), (B) et (C) suivants :
(A) un colorant direct,
(B) un organopolysiloxane modifié par un polyoxyalcylène qui contient de 3 à 30 % en poids d'un groupe polyoxyalcylène dans sa molécule, et qui est dispersible dans l'eau,
(C) un solvant organique,
et présente un pH de 2,0 à 4,5.

**2.** Une composition tinctoriale selon la revendication 1, dans laquelle les proportions-des ingrédients (A), (B) et (C) sont
(A) 0,01 à 5 % en poids,
(B) 0,01 à 10 % en poids et
(C) 0,5 à 50 % en poids
par rapport au poids total de la composition.

**3.** Une composition tinctoriale selon la revendication 1, dans laquelle les proportions des ingrédients (A), (B) et (C) sont
(A) 0,02 à 0,1 % en poids,
(B) 0,1 à 4 % en poids,
(C) 1 à 35 % en poids
par rapport au poids total de la composition.

**4.** Une composition tinctoriale selon l'une quelconque des revendications précédentes, dans laquelle le colorant direct (A) est un colorant acide.

**5.** Une composition tinctoriale selon l'une quelconque des revendications 1 à 3, dans laquelle on choisit le colorant direct (A) dans le groupe formé par le C.I. Acid Yellow 23, le C.I. Solvent Green 7, le C.I. Acid Red 27, le C.I. Acid Red 18, le C.I. Food Green 3, le C.I. Food Blue 2, le C.I. Acid Blue 9, le C.I. Acid Yellow 1, le C.I. Acid Red 52, le C.I. Pigment Red 57-1, le C.I. Acid Orange 7, le C.I. Acid Black 1, le C.I. Acid Green 25 et le C.I. Acid Violet 43.

**6.** Une composition tinctoriale selon l'une quelconque des revendications précédentes, dans laquelle l'organopolysiloxane modifié par un polyoxyalcylène (B) est choisi dans le groupe formé par (B-1), (B-2), (B-3) et (B-4)

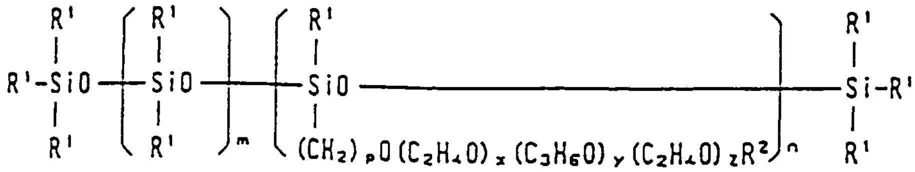

(B-1)

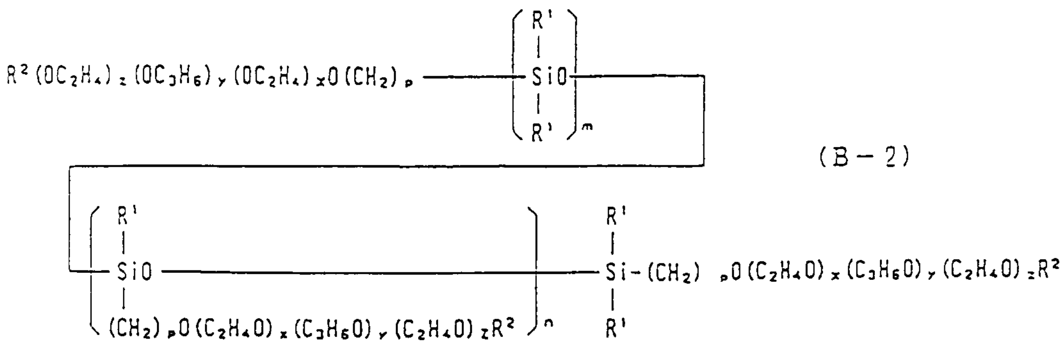

(B-2)

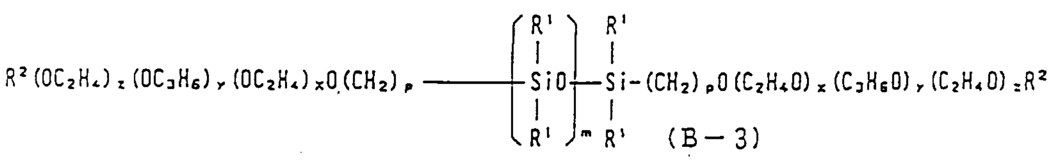

(B-3)

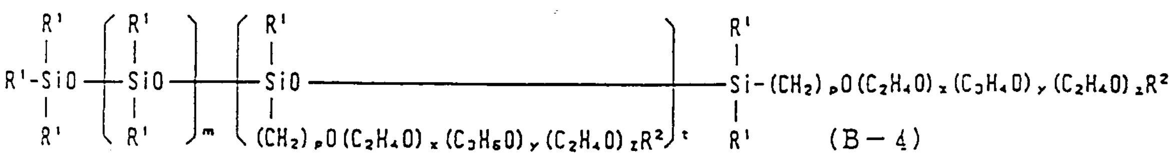

(B-4)

dans lesquelles $R^1$ est un groupe alkyle ou phényle en $C_1$ à $C_3$, $R^2$ est un atome d'hydrogène ou un groupe alkyle en $C_1$ à $C_{12}$, m est un nombre entier de 20 à 200, n est un nombre entier de 1 à 10, x est un nombre entier de 0 à 15, y est un nombre entier de 0 à 10, z est un nombre entier de 0 à 15, p est un nombre entier de 1 à 5, t est un nombre entier de 0 à 10, à condition que la somme de x + y + z soit supérieure ou égale à 1 mais inférieure ou égale à 20.

7. Une composition tinctoriale selon l'une quelconque des revendications précédentes, dans laquelle on choisit le solvant organique (C) dans le groupe formé par les composés représentés par la formule (C-1), les N-alkylpyrrolidones représentés par la formule (C-2), et les carbonates d'alcylène en $C_1$ à $C_4$ :

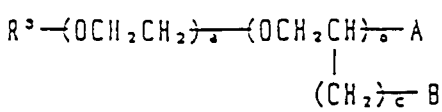

(C-1)

dans laquelle $R^3$ est un atome d'hydrogène, un groupe alkyle inférieur ou un groupe

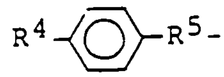

dans laquelle $R^4$ est un atome d'hydrogène, un groupe méthyle ou un groupe méthoxy, $R^5$ est une main pour la liaison d'un hydrocarbone divalent en $C_1$ à $C_3$ saturé ou insaturé, A et B représentent indépendamment un atome d'hydrogène ou un groupe hydroxyle, a, b, et c représentent indépendamment un nombre entier de 0 à 5, sauf les deux cas où a = b = c = 0 et A = H, et a = b = c = 0, $R^3$ = H et A = OH;

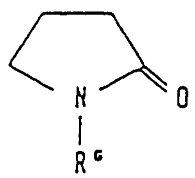 (C − 2)

dans laquelle $R^6$ est un groupe alkyle en $C_1$ à $C_{18}$ linéaire ou ramifié.

**8.** Une composition tinctoriale selon l'une quelconque des revendications 1 à 6, dans laquelle on choisit le solvant organique (C) dans le groupe formé par l'éthanol, l'isopropanol, le n-propanol, le n-butanol, l'isobutanol, l'éthylèneglycol, le propylèneglycol, l'isoprèneglycol, le 1,3-butanediol, l'alcool benzylique, l'alcool cinnamique, l'alcool phénétylique, l'alcool p- anisylique, l'alcool p = méthylbenzylique, le phénoxyéthanol, le 2-benzyloxyéthanol, le méthylcarbitol, l'éthylcarbitol, le propylcarbitol , le butylcarbitol, l'éther monoéthylique de triéthylènegl ycol, l' éther monobutylique de triéthylèneglycol, le glyc érol, la N-méthylpyrrolidone, la N-octylpyrrolidone et la N-lauryl-pyrrolidone.

**9.** Une composition tinctoriale selon l'une quelconque des revendications précédentes, dans laquelle le pH est compris entre 2,5 et 4,5.

**10.** Une composition tinctoriale selon l'une quelconque des revendications précédentes, dans laquelle le pouvoir tampon d'une solution aqueuse à 10 % de ladite composition tinctoriale est de 0,01 à 0,2 équivalent gramme/litre.

**11.** Une composition tinctoriale selon l'une quelconque des revendications précédentes, que l'on applique directement à la chevelure avec les mains nues ou les doigts nus, que l'on laisse reposer de 30 à 300 secondes et que l'on élimine ultérieurement par rinçage avec de l'eau.

**12.** Un procédé de coloration de la chevelure caractérisé en ce que l'on applique une composition comprenant les ingrédients (A) (B) et (C) suivants,

(A) un colorant direct,

(B) un organopolysiloxane modifié par un polyoxyalcylène qui contient de 3 à 30% en poids d'un groupe polyoxyalcylène dans sa molécule, et qui est dispersible dans l'eau,

(C) un solvant organique,

et ayant un pH de 2,0 à 4,5, directement à la chevelure avec les mains nues ou les doigts nus, on laisse reposer pendant 30 à 300 secondes en ensuite' on l'élimine par rinçage avec de l'eau.